(19) **Europäisches Patentamt European Patent Office Office européen des brevets**

(11) **EP 4 734 034 A1**

(12) **EUROPEAN PATENT APPLICATION**
published in accordance with Art. 153(4) EPC

(43) Date of publication:
29.04.2026 Bulletin 2026/18

(21) Application number: 23943560.5

(22) Date of filing: 26.06.2023

(51) International Patent Classification (IPC):
*G06Q 50/00* (2024.01)

(52) Cooperative Patent Classification (CPC):
G06Q 50/00

(86) International application number:
PCT/JP2023/023670

(87) International publication number:
WO 2025/004158 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: FUJITSU LIMITED
Kawasaki-shi, Kanagawa 211-8588 (JP)

(72) Inventors:
• SAWASAKI, Naoyuki
Kawasaki-shi, Kanagawa 211-8588 (JP)
• SASAMOTO, Yuki
Kawasaki-shi, Kanagawa 211-8588 (JP)
• INOMATA, Akihiro
Kawasaki-shi, Kanagawa 211-8588 (JP)

(74) Representative: Hoffmann Eitle
Patent- und Rechtsanwälte PartmbB
Arabellastraße 30
81925 München (DE)

(54) **EXTRACTION METHOD, EXTRACTION PROGRAM, AND INFORMATION PROCESSING DEVICE**

(57)     A computer executes processes of: acquiring a measure including a plurality of conditional branches coupled by a directed edge and each node coupled to a branch destination of each of the plurality of conditional branches; and extracting a conditional branch that is a branch source of each of the nodes from the plurality of conditional branches included in the measure.

FIG.4

## Description

Technical Field

The present invention relates to an extraction method, an extraction program, and an information processing apparatus.

Background Art

As one of workflows, a flow graph is known in which a flow is schematized to allocate target objects of measures, for example, people, in various fields such as medicine, nursing care, and administration to services and the like in order to achieve objectives of the measures.

When measures are planned, there is an aspect in which whether similar measures have been taken in the past is an important consideration from the viewpoint of administrative (political) easiness of execution. Accordingly, the importance of planning measures by incorporating some of measures that have been proven in other regions is increasing.

Citation List

Patent Literature

Patent Document 1: Japanese Laid-open Patent Publication No. 2003-228647

Summary of invention

Technical Problem

Incidentally, when the measures in other regions are applied to an own region, characteristics are different for each region. Therefore, there is an aspect that makes it difficult to directly apply the measures in the other regions to the own region.

On the other hand, it is conceivable to generate an appropriate measure for the own region by combining some measures from a plurality of other regions.

However, design ideas of measures may differ in each region, and measure planners may consider measures on paper. Therefore, it is difficult to extract an element structure of measures.

According to an aspect, an object of the present invention is to provide an extraction method, an extraction program, and an information processing apparatus capable of implementing extraction of element structures of measures.

Solution to Problem

According to an aspect of the embodiment of the invention, an extraction method causes a computer to execute processes of: acquiring a measure including a plurality of conditional branches coupled by a directed edge and each node coupled to a branch destination of each of the plurality of conditional branches; and extracting a conditional branch that is a branch source of each of the nodes from the plurality of conditional branches included in the measure.

Advantageous Effects of Invention

According to an embodiment, it is possible to implement extraction of element structures of measures.

Brief Description of Drawings

FIG. 1 is a block diagram illustrating a functional configuration example of a server apparatus.
FIG. 2 is a diagram illustrating an example of a flow graph of a measure.
FIG. 3 is a diagram illustrating a specific example of a flow graph of a measure.
FIG. 4 is a diagram illustrating one aspect of a problem solving approach.
FIG. 5 is a flowchart illustrating an extraction processing procedure.
FIG. 6 is a diagram illustrating an example of a known measure flow.
FIG. 7 is a schematic diagram illustrating an extraction example of a path structure.
FIG. 8 is a diagram illustrating an example of a correspondence relationship between a parameter type and a code.
FIG. 9 is a diagram illustrating an example of a data structure related to a path structure.

FIG. 10 is a flowchart illustrating a generation processing procedure.
FIG. 11 is a schematic diagram illustrating an example of a list structure.
FIG. 12 is a schematic diagram illustrating a generation example of a measure candidate flow.
FIG. 13 is a diagram illustrating clustering of branch thresholds and branch probabilities.
FIG. 14 is a flowchart illustrating a procedure of a branch threshold setting process.
FIG. 15 is a diagram illustrating a hardware configuration example.

Description of Embodiments

Hereinafter, embodiments of an extraction method, an extraction program, and an information processing apparatus according to the present application will be described with reference to the accompanying drawings. In each embodiment, only one example and aspect are illustrated. A numerical value, a function range, a use scene, and the like are not limited by such an example. The embodiments can be appropriately combined within a range in which the processing content does not contradict each other.

<First Embodiment>

<System Configuration>

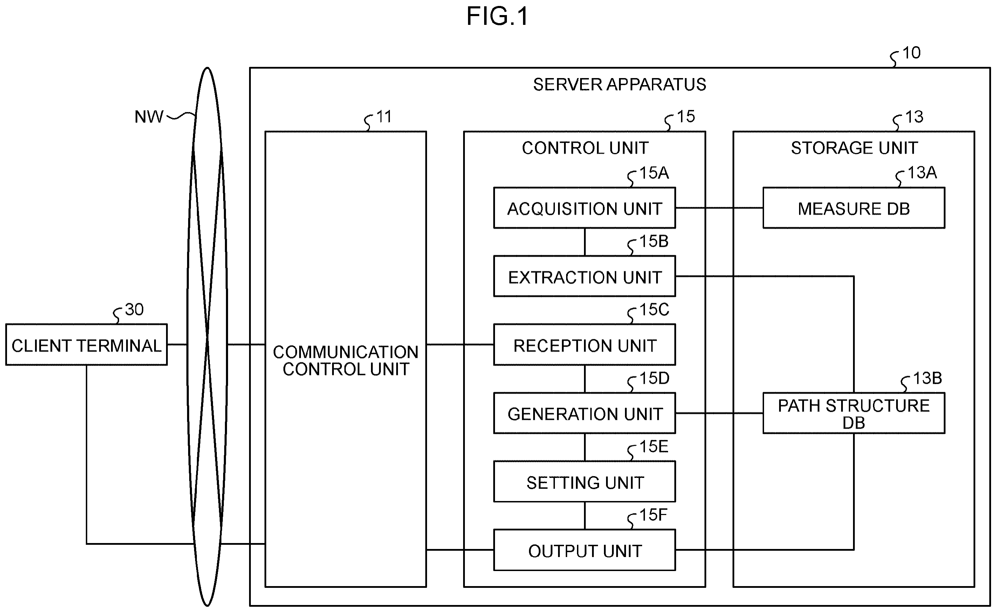

FIG. 1 is a block diagram illustrating a functional configuration example of a server apparatus 10. The server apparatus 10 illustrated in FIG. 1 provides a data infrastructure platform capable of enabling sharing, cross-referencing, and updating of a flow graph of measures.
For example, the server apparatus 10 can provide a function of a platform of the above-described data infrastructure as a cloud service by executing middleware of a platform as a service (PaaS) or an application of a software as a service (SaaS). The server apparatus 10 corresponds to an example of an information processing apparatus.
As illustrated in FIG. 1, the server apparatus 10 can be communicably connected to a client terminal 30 via a network NW. For example, the network NW may be any type of communication network such as the Internet or a local area network (LAN) regardless of whether the network NW is wired or wireless. FIG. 1 illustrates an example in which one client terminal 30 is connected to one server apparatus 10, but any number of client terminals 30 is not precluded from being connected.
The client terminal 30 is a terminal apparatus that receives provision of a data infrastructure. For example, the client terminal 30 can be used by a measure planner as an example of a person involved in implementing a measure, for example, a local government. As an example, the client terminal 30 may be implemented by any computer such as a personal computer, a smartphone, a tablet terminal, or a wearable terminal.

<Flow Graph of Measures>

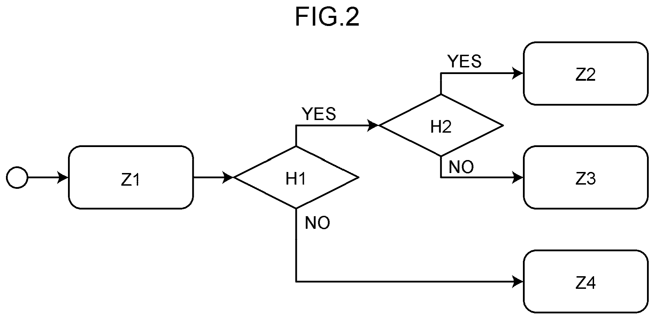

A flow graph of the above measures is illustrated in FIG. 2. FIG. 2 is a diagram illustrating an example of a flow graph of a measure. Z1, Z2, Z3, and Z4 illustrated in FIG. 2 indicate, for example, services provided by an administrator to a user. These services may be referred to as "service implementation components". Specific examples of the services include, for example, "intervention" in which a target object of measures such as a medical examination or an examination by a specialist, for example, a resident or the like, is assigned, and "no intervention" such as follow-up, but are not limited to measures in the medical field.
H1 and H2 indicate, for example, conditional branches including conditions. These components may be referred to as "conditional branch components". Specific examples of the conditions include, for example, in the medical field, an estimated glemerular filtration rate (eGFR) is less than the threshold, a hemoglobin A1c value (HbA1c) is less than a threshold, and a urinary protein value is equal to or more than a threshold, but are not limited to the conditions in the medical field.
Z1, Z2, Z3, Z4 and H1, H2 may each be referred to as "components". Such a "component" can correspond to an example of a "node" in terms of graph data. Hereinafter, of the nodes, a node corresponding to a branch may be referred to as a "branch node", and a node corresponding to a service (intervention) for achieving a purpose of a measure may be referred to as a "service node". Connection between nodes can correspond to an example of an "edge" including a "directed edge" and the like.
In the present embodiment, planning of measures in the medical field will be described as an example, but the present invention is not limited thereto. The above-described embodiment may be used for various measures such as work having conditional branches, tests, and questionnaires. In this case, the same operations and effects as those of the above-described embodiment can be obtained.

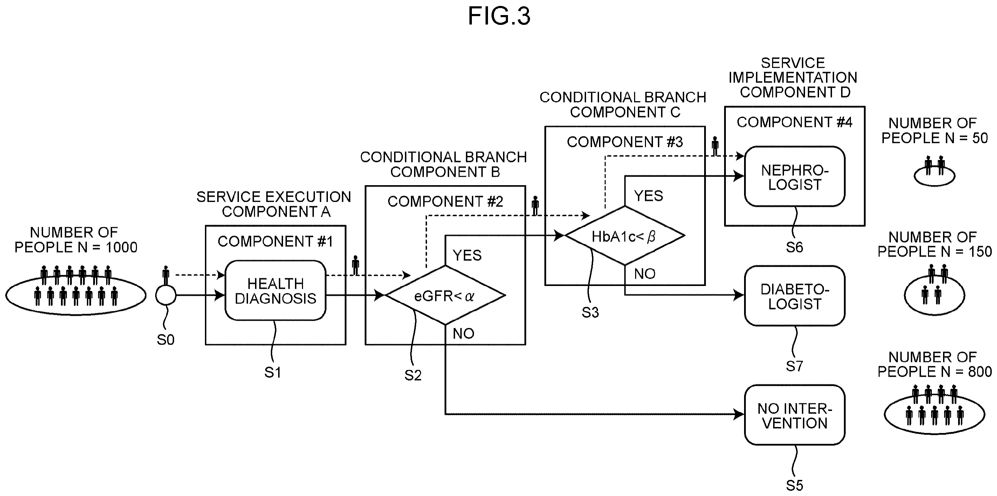

FIG. 3 is a diagram illustrating a specific example of a flow graph of a measure. As illustrated in FIG. 3, a measure is modeled as a workflow including a combination of components such as conditional branches and service implementation. Then, the number of people who receive each service is output from the model trained by accumulating information and parameters of a flow of people from actual values at the time of use for conditional branch components. Accordingly, the local government can adopt a measure appropriate for the local government among the measures implemented by other local governments in consideration of resources of the local government.

In the example illustrated in FIG. 3, the number of people N = 1000 is input at reference numeral S0. In reference numeral S1, component #1 serving as a service implementation component A is set in "medical examination". In reference numeral S2, component #2 serving as a conditional branch component B is set in "eGFR<$\alpha$". In a case where "eGFR<$\alpha$" is not satisfied (see NO route of reference numeral S2), it is determined that "there is no intervention" by the specialist for the citizen as denoted by reference numeral S5.

Conversely, when "eGFR<$\alpha$" is satisfied (see YES route of reference numeral S2), component #3 serving as a conditional branch component C is set in "HbA1c<$\beta$" as denoted by reference numeral S3. When "HbA1c<$\beta$" is satisfied (see YES route of reference numeral S3), as denoted by reference numeral S6, component #4 serving as a conditional branch component D is set in "nephrologist", and it is determined that intervention of "nephrologist" is needed for the citizen. Conversely, when "HbA1c<$\beta$" is not satisfied (see NO route of reference numeral S3), as denoted by reference numeral S7, it is determined that intervention of "diabetologist" is needed for the citizen.

In the example illustrated in FIG. 3, as indicated by arrows, the number of people flowing through components #1, #2, #3, and #4 in this order is predicted. For example, in the flow graph of the measure illustrated in FIG. 3, a result obtained by allocating the number of people N = 1000 to the intervention Z2 to Z4 is as follows. Fifty people are assigned to intervention Z2. One hundred fifty people are assigned to intervention Z3. Eight hundred people are assigned to intervention Z4.

Here, a specific example of another use of the flow graph of the measure will be described with reference to FIGS. 2 and 3. The server apparatus 10 retrieves the flow graph of the measure using attribute information of a person of an institution to which a measure planner belongs. The server apparatus 10 specifies a node into which a person is classified among nodes located at ends that form the flow graph of the measure. Accordingly, the local government to which the measure has been applied can specify a medical institution to be recommended to the person in consideration of a health state of a person belonging to the local government and the resources of the local government.

First, the server apparatus 10 specifies a person included in the institution to which the measure planner belongs. For example, the server apparatus 10 specifies a resident of the local government. Subsequently, the server apparatus 10 specifies a node in which the specified person is classified among the nodes located at the ends that form the flow graph of the measure by retrieving the output flow graph of the measure using the attribute information of the person of the institution to which the measure planner belongs. The attribute information is biological information specified by analyzing body fluids of the person. The attribute information is an estimated glomerular filtration rate, a hemoglobin A1c value, a Urinary protein value, and the like. The body fluids include blood, a lymph fluid, a tissue fluid (an interstitial fluid, an intercellular fluid, an interstitial fluid) sweat, tears, nasal discharge, urine, semen, a vaginal fluid, an amniotic fluid, and breast milk.

At this time, the server apparatus 10 specifies the node in which the person is classified by comparing the attribute information of the person with a condition included in the conditional branch component. The server apparatus 10 specifies a node in which the specified person is classified among the nodes located at the ends. Then, the server apparatus 10 sets a medical institution indicated by the specified classified node as a medical institution to be recommended to the specified person. The medical institution indicated by the node is a nephrologist, a diabetologist, or the like.

Hereinafter, a measure that has already been implemented and has a track record may be referred to as a "known measure", and a measure that is listed as an option to be implemented at the time of planning the measure may be referred to as a "measure candidate". Further, a flow graph of a measure may be abbreviated as a "measure flow". In addition, a measure flow corresponding to a known measure in the measure flow may be referred to as a "known measure flow", and a measure flow corresponding to a measure candidate may be referred to as a "measure candidate flow".

<Data Infrastructure>

In the above data infrastructure, a measure flow may be shared in any framework. The above data infrastructure that is merely exemplary can share a measure flow between institutions in the world, for example, public institutions such as local governments.

The measure planner can refer to templates of known measures in the world shared in the above-described data infrastructure through the client terminal 30. For example, from the viewpoint of administrative (political) ease of execution, a measure candidate can be generated by incorporating a part of each of flow graphs of a plurality of known measures among templates collected in a data infrastructure. The flow graph of such a measure candidate may be automatically or manually generated using any technique.

<One Aspect of Problem>

As described in Background described above, when the measures in other regions are applied to an own region, characteristics are different for each region. Therefore, there is an aspect that makes it difficult to directly apply the measures in other regions to the own region.

On the other hand, it is conceivable to generate an appropriate measure for the own region by combining some measures from a plurality of other regions.

However, design ideas of measures may differ in each region, and measure planners may consider measures on paper. Therefore, it is difficult to extract an element structure of measures.

<One Aspect of Problem Solving Approach>

Accordingly, in the present example, for each service included in the flow graph of the known measures collected in the data infrastructure, an extraction function for extracting an array of conditions corresponding to a path formed by connecting branch nodes up to a terminal node corresponding to the service as a path structure is mounted.

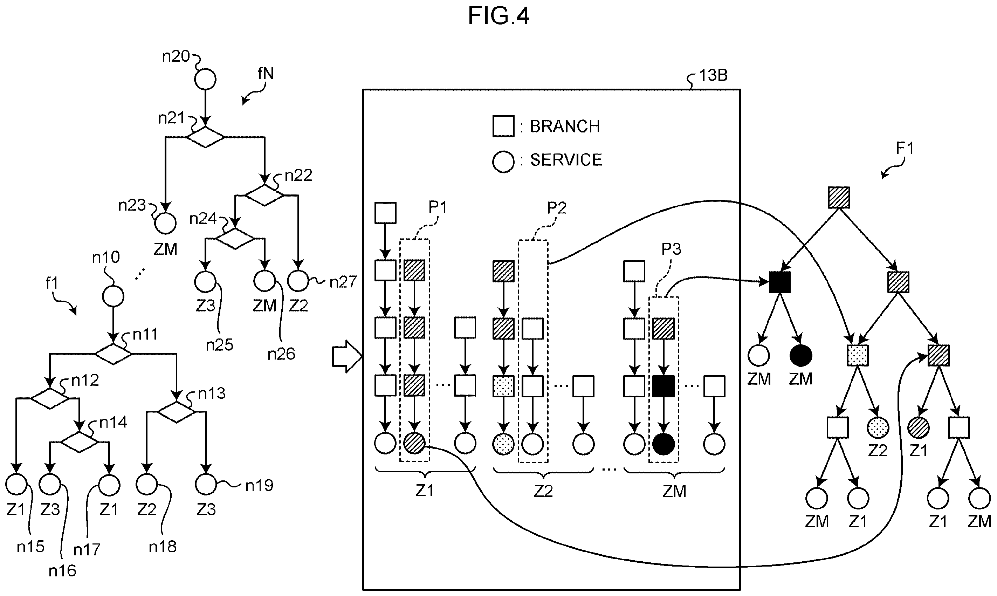

FIG. 4 is a diagram illustrating one aspect of the problem solving approach. As illustrated in FIG. 4, N known measure flows f1 to fN are collected in the data infrastructure. For each service included in each of the N known measure flows f1 to fN, an array of conditions corresponding to a path formed by connecting branch nodes up to a terminal node corresponding to the service is extracted.

For example, in the case of the service Z1 (node n15) included in the known measure flow f1, an array of conditions corresponding to a path continuing from the node n10 to the node n15 via the nodes n11 and n12 is extracted. Similarly, an array of conditions corresponding to a path of the service Z3 (node n16), a path of the service Z1 (node n17), a path of the service Z2 (node n18), and a path of the service Z3 (node n19) included in the known measure flow f1 is extracted.

In the case of the service ZM (node n23) included in the known measure flow fN, an array of conditions corresponding to a path formed from the node n20 to the node n23 via the node n21 is extracted. Similarly, an array of conditions corresponding to a path of the service Z3 (node n25), a path of the service ZM (node n26), and a path of the service Z2 (node n27) included in the known measure flow fN is extracted.

Accordingly, in the extraction function according to the present embodiment, it is possible to implement extraction of a path structure as an example of an element structure of a known measure.

In this way, by retrieving a set of paths corresponding to a service scheduled to be provided at the time of implementing the measure from a path structure database (DB) 13B that stores a set of path structures extracted for each service, it is possible to generate a measure candidate flow.

As a mere example, when the services Z1, Z2, and ZM are designated as services scheduled to be provided at the time of implementing the measure, the measure candidate flow F1 can be generated by retrieving the path P1 of the service Z1, the path P2 of the service Z2, and the path P3 of the service ZM from the path structure DB 13B and combining these paths.

By extracting the path structure in units of services of the known measure flow in this way, it is possible to generate a measure candidate flow by incorporating some paths appropriate for region characteristics of a draft target from each of the flow graphs of the plurality of known measures.

<Configuration of Server Apparatus 10>

FIG. 1 schematically illustrates blocks related to a data infrastructure included in the server apparatus 10. As illustrated in FIG. 1, the server apparatus 10 includes a communication control unit 11, a storage unit 13, and a control unit 15. FIG. 1 merely illustrates excerpted functional units related to the above-described data infrastructure, and functional units other than those illustrated may be provided in the server apparatus 10.

The communication control unit 11 is a functional unit that controls communication with another apparatus such as the client terminal 30. As a mere example, the communication control unit 11 can be implemented by a network interface card such as a LAN card. As one aspect, the communication control unit 11 receives a measure generation request for requesting generation of a measure candidate flow from the client terminal 30, or outputs a generation result of the measure candidate flow to the client terminal 30.

The storage unit 13 is a functional unit that stores various types of data. As a mere example, the storage unit 13 is implemented by an internal, external, or auxiliary storage of the server apparatus 10. For example, the storage unit 13 stores a measure DB 13A that stores a set of measure flows and a path structure DB 13B that stores a set of path structures.

The control unit 15 is a functional unit that executes overall control of the server apparatus 10. For example, the control unit 15 can be implemented by a hardware processor. In addition, the control unit 15 may be implemented by hard-wired logic. As illustrated in FIG. 1, the control unit 15 includes an acquisition unit 15A, an extraction unit 15B, a reception unit 15C, a generation unit 15D, a setting unit 15E, and an output unit 15F.

The acquisition unit 15A is a processing unit that acquires a measure flow. As a mere example, the acquisition unit 15A acquires K (any natural number) known measure flows stored in the measure DB 13A. Here, an example in which the known measure flow is acquired from the measure DB 13A has been described. However, the known measure flow may be acquired from the outside via the network NW, or the known measure flow may be acquired from removable media (not illustrated).

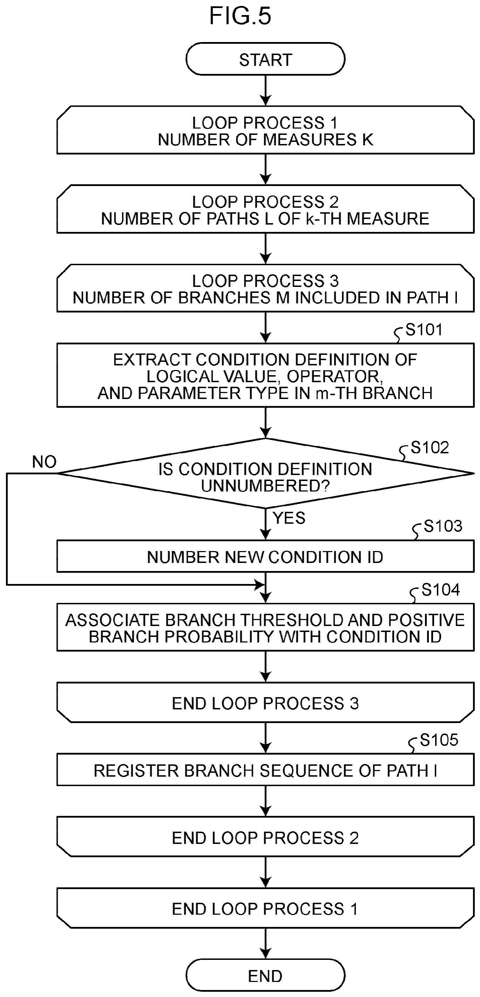

The extraction unit 15B is a processing unit that extracts the above-described path structure. FIG. 5 is a flowchart illustrating an extraction processing procedure. As illustrated in FIG. 5, the extraction unit 15B executes a loop process 1 in which processes from the following step S101 to the following step S105 are repeated by the number of times corresponding to a number K of known measure flows acquired by the acquisition unit 15A. Further, the extraction unit 15B executes a loop process 2 in which processes from the following step S101 to the following step S105 are repeated by the number of times corresponding to a number L of paths included in a k-th known measure flow. In addition, the extraction unit 15B executes a loop process 3 in which processes from the following step S101 to the following step S104 are repeated by the number of times corresponding to a number M of branches included in the path l of the k-th known measure flow.

That is, the extraction unit 15B extracts a condition definition including a logical value, an operator, and a parameter type from an m-th branch included in the path l of the k-th known measure flow (step S101). At this time, when the condition ID of the condition definition extracted in step S101 is unnumbered (YES in step S102), the extraction unit 15B numbers a new condition ID (step S103). When the condition ID of the condition definition extracted in step S101 is not unnumbered (NO in step S102), the process in step S103 is skipped.

Thereafter, the extraction unit 15B registers the branch threshold and the positive branch probability in the m-th branch included in the path l in association with the condition ID corresponding to the condition definition extracted in step S101 (step S104). The "branch probability" mentioned herein indicates a probability that an object is allocated to a branch destination corresponding to a logical value by a branch threshold at a branch of a path.

By repeating the loop process 3, condition definitions, for example, logical values, operators, parameter types, and the like are extracted for every M branches included in the path l of the k-th known measure flow.

Then, the extraction unit 15B registers an array of M branch condition definitions included in the path l of the k-th known measure flow in the path structure DB 13B as a branch sequence of the path l (step S105).

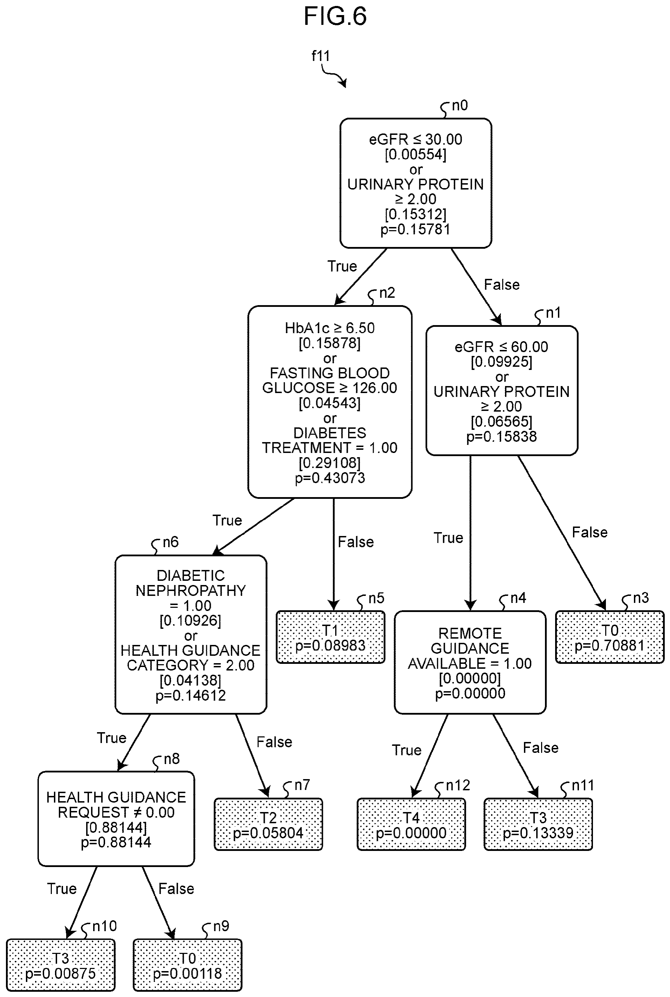

FIG. 6 is a diagram illustrating an example of a known measure flow. FIG. 6 illustrates a known measure flow f11 related to medical care follow-up of chronic kidney disease (CKD) as a mere example. As illustrated in FIG. 6, the known measure flow f11 includes path #1 continuing from the node n0 to the node n10 of a service "health guidance" via the nodes n2, n6, and n8. Further, the known measure flow f11 includes path #2 continuing from the node n0 to the node n11 of the service "health guidance" via the nodes n1 and n4.

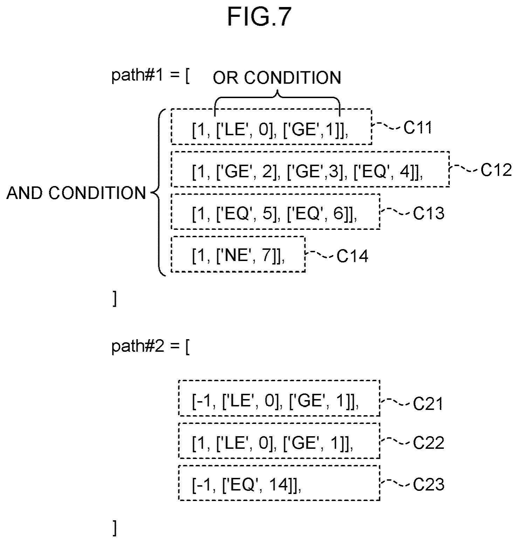

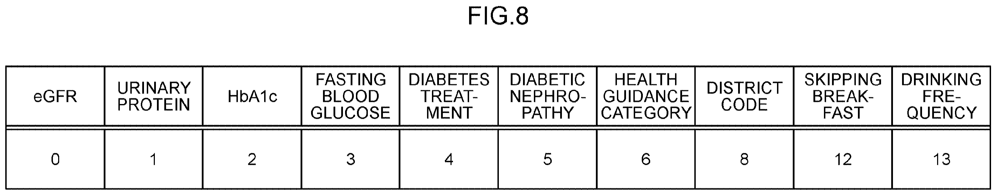

FIG. 7 is a schematic diagram illustrating an extraction example of a path structure. FIG. 7 illustrates an example in which the structures of paths #1 and #2 of the known measure flow f11 illustrated in FIG. 6 are extracted. First, an example in which a condition definition C11 of the branch node n0 of path #1 is extracted will be described. As illustrated in FIG. 7, since path #1 is a route corresponding to a branch destination of a logical value "True" of the branch node n0, a logical value code "1" associated with the logical value "True" is extracted. Then, an operator code "LE" corresponding to an operator "$\leq$ (or less)" is extracted from the first condition "eGFR $\leq$ 30.0" among the OR conditions of the branch node n0. Further, a parameter type code corresponding to the parameter type "eGFR" is extracted from the first condition "eGFR $\leq$ 30.0" of the branch node n0. These parameter types and codes are expressed in a correspondence relationship illustrated in FIG. 8. FIG. 8 is a diagram illustrating an example of a correspondence relationship between a parameter type and a code. As illustrated in FIG. 8, since the parameter type "eGFR" of the first condition "eGFR $\leq$ 30.0" of the branch node n0 corresponds to a parameter type code "0", the parameter type code "0" is extracted. The operator code "GE" corresponding to the operator "$\geq$ (or more)" is extracted from the second condition "urinary protein e$\geq$2.00" among the OR conditions of the branch node n0. Further, the parameter type code "1" corresponding to the parameter type "urinary protein" is extracted from the second condition "urinary protein e $\geq$ 2.00" of the branch node n0. Through the series of processes, the condition definition C11 of the branch node n0 of path #1 is extracted.

Next, an example in which the condition definition C12 of the branch node n2 of path #1 is extracted will be described. As illustrated in FIG. 7, since path #1 is a route corresponding to the branch destination of the logical value "True" of the branch node n2, the logical value code "1" associated with the logical value "True" is extracted. The operator code "GE" corresponding to the operator "$\geq$ (or more)" is extracted from the first condition "HbA1c $\geq$ 6.50" among the OR conditions of the branch node n2. Further, a parameter type code "2" corresponding to the parameter type "HbA1c" is extracted from the first condition "HbA1c $\geq$ 6.50" of the branch node n2. The operator code "GE" corresponding to the operator "$\geq$ (or more)" is extracted from the second condition "fasting blood glucose $\geq$ 126.00" among the OR conditions of the branch node n2. Further, a parameter type code "3" corresponding to the parameter type "fasting blood glucose" is extracted from the second condition "fasting blood glucose $\geq$ 126.00" of the branch node n2. Further, an operator code "EQ" corresponding to the operator "=" is extracted from the third condition "diabetes treatment = 1.00" of the OR condition of the branch node n2. Further, a parameter type code "4" corresponding to the parameter type "diabetes treatment" is extracted from the third condition "diabetes treatment = 1.00" of the branch node n2. Through the series of processes, the condition definition C12 of the branch node n2 of path #1 is extracted.

Next, an example in which the condition definition C13 of the branch node n6 of path #1 is extracted will be described. As illustrated in FIG. 7, since path #1 is a route corresponding to the branch destination of the logical value "True" of the branch node n6, the logical value code "1" associated with the logical value "True" is extracted. Then, the operator code "EQ" corresponding to the operator "=" is extracted from the first condition "diabetic nephropathy = 1.00" of the OR condition of the branch node n6. Further, a parameter type code "5" corresponding to the parameter type "diabetic nephropathy" is extracted from the first condition "diabetic nephropathy = 1.00" of the branch node n6. The operator code "EQ" corresponding to the operator "=" is extracted from the second condition "health guidance category = 2.00" of the OR condition of the branch node n6. Further, a parameter type code "6" corresponding to the parameter type "health guidance category" is extracted from the second condition "health guidance category = 2.00" of the branch node n6. Through the series of processes, the condition definition C13 of the branch node n6 of path #1 is extracted.

Finally, an example in which the condition definition C14 of the branch node n8 of path #1 is extracted will be described. As illustrated in FIG. 7, since path #1 is a route corresponding to the branch destination of the logical value "True" of the branch node n8, the logical value code "1" associated with the logical value "True" is extracted. Then, an operator code "NE" corresponding to an operator "$\neq$" is extracted from the condition "health guidance request $\neq$ 0.00" of the branch node n8. Further, a parameter type code "7" corresponding to the parameter type "health guidance request" is extracted from the condition "health guidance request $\neq$ 0.00" of the branch node n8. Through the series of processes, the condition definition C14 of the branch node n8 of path #1 is extracted.

As described above, the array of the condition definition C11, the condition definition C12, the condition definition C13, and the condition definition C14 corresponding to path #1 are extracted as a branch sequence of path #1.

Further, similarly to path #1, an array of three condition definitions corresponding to path #2 can be extracted as a branch sequence of path #2. First, an example in which the condition definition C21 of the branch node n0 of path #2 is extracted will be described. As illustrated in FIG. 7, since path #2 is a route corresponding to the branch destination of the logical value "False" of the branch node n0, a logical value code "-1" associated with the logical value "False" is extracted. Then, an operator code "LE" corresponding to an operator "$\leq$ (or less)" is extracted from the first condition "eGFR $\leq$ 30.0" among the OR conditions of the branch node n0. Further, the parameter type code "0" corresponding to the parameter type "eGFR" is extracted from the first condition "eGFR $\leq$ 30.0" of the branch node n0. The operator code "GE" corresponding to the operator "$\geq$ (or more)" is extracted from the second condition "urinary protein e$\geq$2.00" among the OR conditions of the branch node n0. Further, the parameter type code "1" corresponding to the parameter type "urinary protein" is extracted from the second condition "urinary protein e $\geq$ 2.00" of the branch node n0. Through the series of processes, the condition definition C21 of the branch node n0 of path #2 is extracted.

Next, an example in which a condition definition C22 of the branch node n1 of path #2 is extracted will be described. As illustrated in FIG. 7, since path #2 is a route corresponding to the branch destination of the logical value "True" of the branch node n1, the logical value code "1" associated with the logical value "True" is extracted. The operator code "LE" corresponding to the operator "$\leq$ (or less)" is extracted from the first condition "eGFR $\leq$ 60.0" of the OR conditions of the branch node n1. Further, the parameter type code "0" corresponding to the parameter type "eGFR" is extracted from the first condition "eGFR $\leq$ 60.0" of the branch node n1. The operator code "GE" corresponding to the operator "$\geq$ (or more)" is extracted from the second condition "urinary protein e $\geq$ 2.00" among the OR conditions of the branch node n1. Further, a parameter type code "1" corresponding to the parameter type "urinary protein" is extracted from the second condition "urinary protein e $\geq$ 2.00" of the branch node n1. Through the series of processes, the condition definition C22 of the branch node n1 of path #2 is extracted.

Finally, an example in which a condition definition C23 of the branch node n4 of path #2 is extracted will be described. As illustrated in FIG. 7, since path #2 is a route corresponding to the branch destination of the logical value "False" of the branch node n4, the logical value code "-1" associated with the logical value "False" is extracted. The operator code "EQ" corresponding to the operator "=" is extracted from the condition "remote instruction available = 1.00" of the branch node n4. Further, the parameter type code "14" corresponding to a parameter type "remote guidance available" is extracted from a condition "remote guidance available = 1.00" of the branch node n4. Through the series of processes, the condition definition C23 of the branch node n4 of path #2 is extracted.

As described above, the array of the condition definition C21, the condition definition C22, and the condition definition C23 corresponding to path #2 are extracted as the branch sequence of path #2.

Here, the above-described path structure is structured and registered for each service in step S105 illustrated in FIG. 5. That is, the extraction unit 15B registers an array of condition definitions corresponding to a path formed by connecting branch nodes up to a terminal node corresponding to a service is registered as a branch sequence.

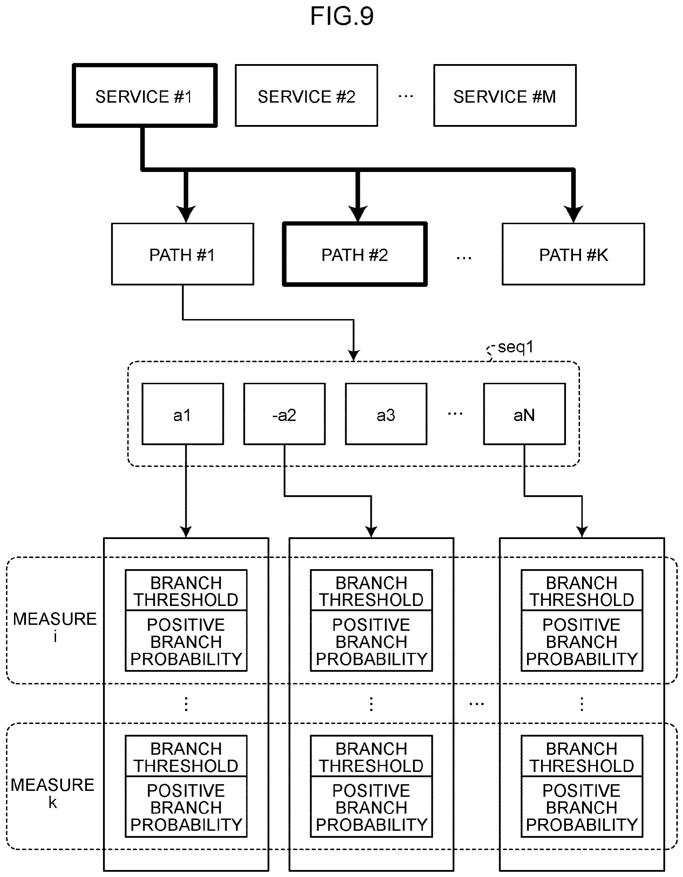

FIG. 9 is a diagram illustrating an example of a data structure related to a path structure. As illustrated in FIG. 9, path #1, paths #2,..., and path #K formed by connecting branch nodes up to the terminal node corresponding to service #1 are associated with service #1. Further, in association with path #1, an array of the condition definition of the condition ID "a1", the condition definition of the condition ID "-a2", the condition definition of the condition ID "a3",..., and the condition definition of the condition ID "aN" is registered as a branch sequence seq1. A branch threshold and a positive branch probability of one or a plurality of measures are registered in the condition ID "a1", the condition ID "-a2", the condition ID "a3",..., and the condition ID "aN" in association. In the example illustrated in FIG. 9, the condition definition of the condition ID "a1", the condition definition of the condition ID "-a2", and the condition definition of the condition ID "aN" are common between measures i and k, but the branch threshold and the positive branch probability are individually registered for each of the measures i and k.

The extracted paths, that is, the array of the condition definitions (condition IDs), can also be displayed in order corresponding to a directed edge, for example, in hierarchical order of a tree structure. At this time, when the threshold of the condition in the branch is displayed, a statistical value in each measure illustrated in FIG. 9, for example, an average, a median, or the like, can be displayed.

Referring back to FIG. 1 for description, the reception unit 15C is a processing unit that receives various requests from the client terminal 30. As a mere example, the reception unit 15C can receive a measure generation request for requesting generation of a measure candidate flow from the client terminal 30. The measure generation request may include designation of one or more services to be provided at the time of implementing the measure as a mere example. Hereinafter, a service scheduled to be provided at the time of implementing the measure may be referred to as a "provision-scheduled service".

The generation unit 15D is a processing unit that generates a measure candidate flow. As a mere example, the generation unit 15D enumerates condition IDs to be allocated to branches included in the path of the measure candidate flow in order from a start point of the path of the measure candidate flow according to a branch sequence of the provision-scheduled service among branch sequences included in the path structure DB 13B.

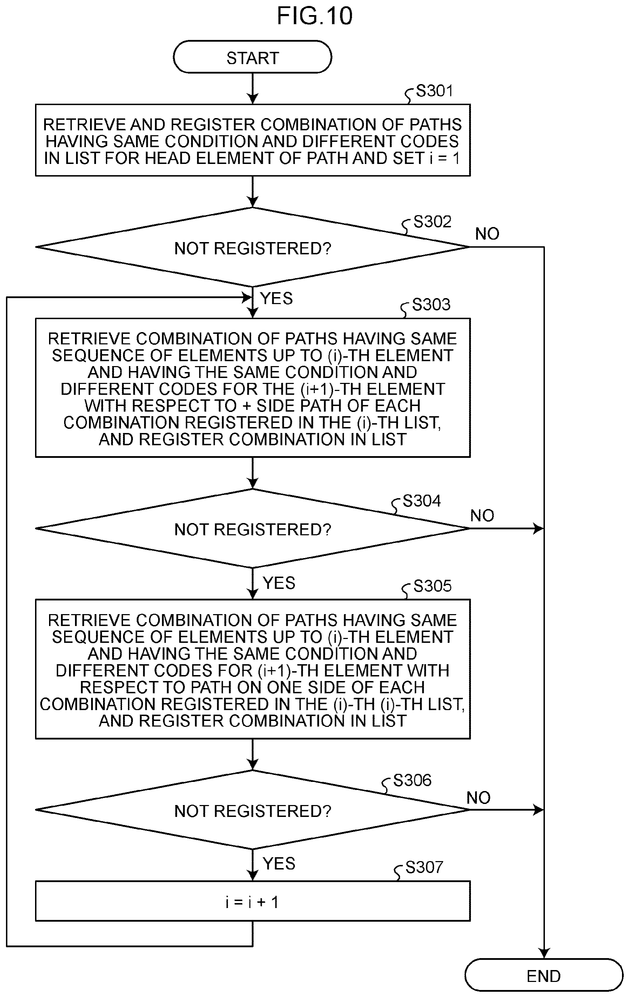

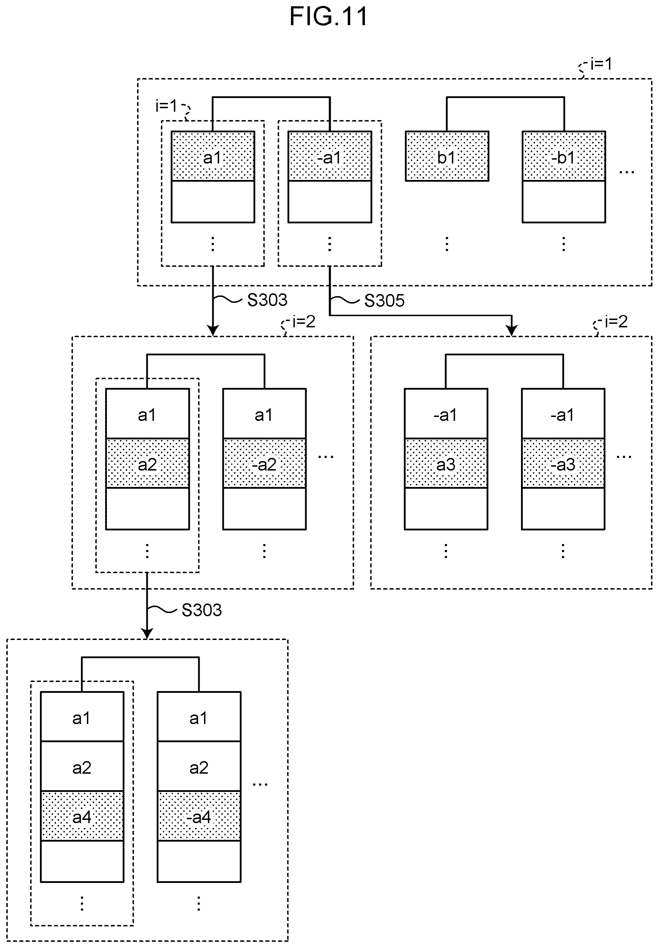

FIG. 10 is a flowchart illustrating a generation processing procedure. FIG. 11 is a schematic diagram illustrating an example of the list structure. The process illustrated in FIG. 10 is executed as a mere example when the reception unit 15C receives a measure generation request.

As illustrated in FIG. 10, the generation unit 15D executes the process of step S301 for a head element of the path of a measure candidate flow to be generated.

That is, the generation unit 15D retrieves a pair of two condition IDs having the same operator and the same parameter type and having different signs of logical values among the condition definitions at the head of the branch sequence corresponding to the provision-scheduled service among the branch sequences included in the path structure DB 13B. Further, as illustrated in FIG. 11, the generation unit 15D registers a pair of two condition IDs obtained by the retrieval of step S301 in a list, and sets an index i for identifying a depth in a tree structure of the list, that is, a so-called hierarchy, to "1".

Subsequently, when there is no pair of condition IDs to be registered in the list of i = 1 from the path structure DB 13B (YES in step S302), the generation unit 15D executes the process of step S303.

That is, the generation unit 15D retrieves a pair of two condition IDs matching the array of condition IDs in which a sign of a logical value of an i-th condition ID is "positive" in the array of condition IDs registered in the i-th list up to an i-th hierarchy from a start point to the i-th hierarchy, that is, a hierarchy at the depth where the pairs of condition IDs have already been listed, having the same operator and same parameter type in (i+1)-th condition definition, and having different signs of the logical value, from the branch sequence corresponding to the provision-scheduled service. Then, as illustrated in FIG. 11, the generation unit 15D registers a pair of two condition IDs obtained through the retrieval of step S303 in the list.

When the array of condition IDs matching the array of condition IDs of which the sign of the logical value of the i-th condition ID is "positive" disappears from the path structure DB 13B (YES in step S304), the generation unit 15D executes the process of step S305.

That is, the generation unit 15D retrieves a pair of two condition IDs matching an array of condition IDs in which a sign of a logical value of the i-th condition ID is "negative" in the array of condition IDs registered in the i-th list up to the i-th hierarchy from the start point to the i-th hierarchy, that is, the hierarchy at the depth where the pairs of condition IDs have already been listed, having the same operator and same parameter type in the (i+1)-th condition definition, and having different signs of the logical value, from the branch sequence corresponding to the provision-scheduled service. Then, as illustrated in FIG. 11, the generation unit 15D registers a pair of two condition IDs obtained through the retrieval of step S305 in the list.

Thereafter, when the array of condition IDs matching the array of condition IDs of which the sign of the logical value of the i-th condition ID is "negative" disappears from the path structure DB 13B (YES in step S306), the generation unit 15D executes the following process. That is, the generation unit 15D increases the index i for identifying the hierarchy of the list (step S307), and then the process proceeds to step S303.

The processes from step S303 to step S307 are repeatedly executed until the list is no longer registered. Accordingly, a graph structure of the measure candidate flow F in the array of condition IDs is listed as an instance. At this time, whenever a plurality of candidates are retrieved as a pair of condition IDs whose signs of logical values are inverted, a measure candidate flow having a different array of condition IDs is obtained by a number corresponding to the number of candidates.

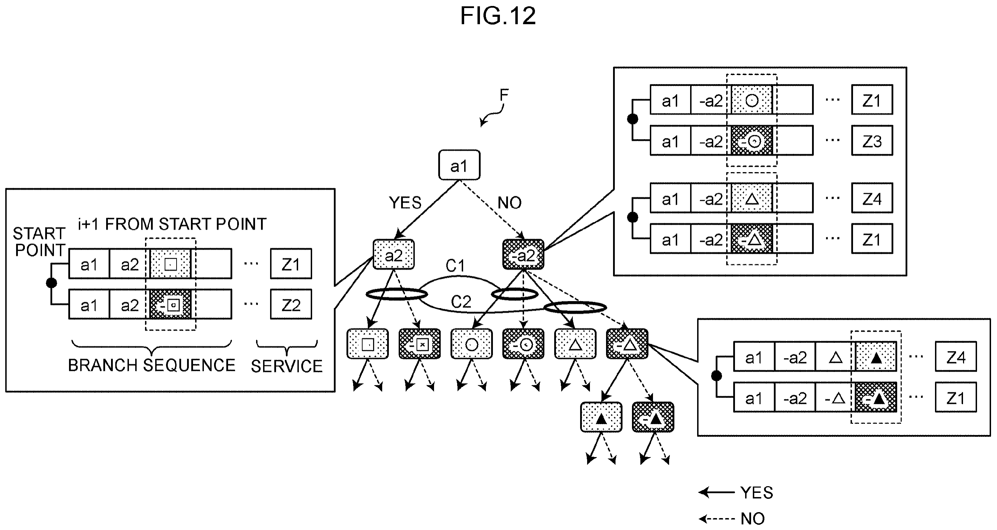

FIG. 12 is a schematic diagram illustrating a generation example of a measure candidate flow. As illustrated in FIG. 12, among the branch sequences included in the path structure DB 13B, comparison is executed in order from the head of the branch sequence corresponding to the provision-scheduled service and possible combinations of positive and negative branches are stored as a tree structure. For example, a pair of condition IDs in which the branch sequence from a start point to an i-th branch is matched and a sign of the logical value in the (i+1)-th branch is inverted between positive and negative signs is retrieved and then stored as a list. In this way, combinations of possible condition IDs from the tip of the tree structure are listed, and then the possible condition ID to which a service is assigned is selected at a terminal. At this time, the graph structure of the measure candidate flow F is listed as an instance by duplicating the array of condition IDs by the number of candidates whenever a plurality of candidates, C1 and C1 in the example of FIG. 12, are retrieved as a pair of condition IDs in which the signs of logical values are inverted, that is, C1 and C1 are retrieved.

As described above, when a plurality of nodes are designated as the provision-scheduled service, the generation unit 15D generates a measure candidate flow including a plurality of designated nodes and conditional branches corresponding to the plurality of designated nodes with reference to the path structure DB 13B that stores each node and the extracted condition of the branch source in association.

Referring back FIG. 1 for description, the setting unit 15E is a processing unit that sets a threshold in a branch condition included in the measure candidate flow. Here, the measure candidate flow generated by the generation unit 15D is an instance of a graph structure in which a plurality of paths are combined, and no threshold is set for a condition of a branch of each path at this time point.

As illustrated in FIG. 9, the condition ID in such a branch can use the branch threshold and the branch probability extracted for each measure at the time of extracting the path structure for setting the threshold of the measure candidate flow. However, when there are many variations in the branch threshold, there is an aspect that the number of possible combinations becomes enormous.

From this aspect, the setting unit 15E can obtain a cluster of the threshold and the probability by modeling and clustering a simultaneous distribution of the branch threshold and the branch probability for the branch of the measure candidate flow generated by the generation unit 15D.

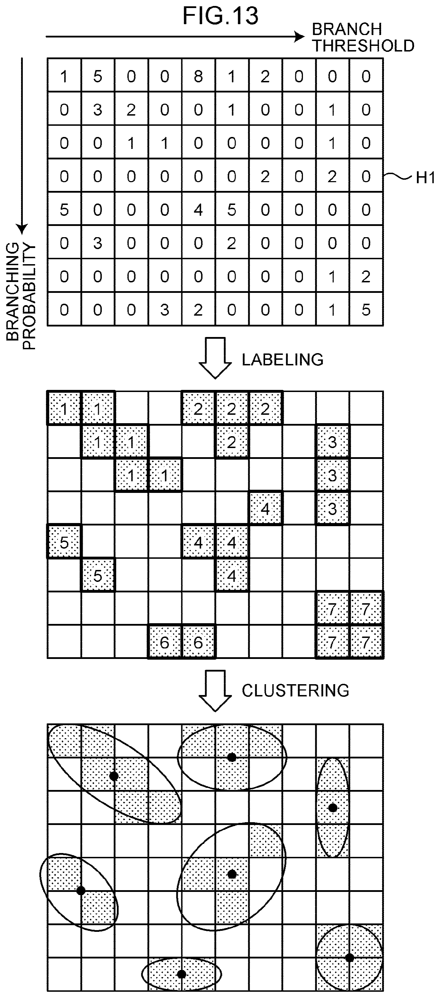

FIG. 13 is a diagram illustrating clustering of branch thresholds and branch probabilities. As illustrated in FIG. 13, the setting unit 15E generates a two-dimensional histogram H1 by mapping each of the branch threshold and the branch probability data associated with the condition ID of the branch of the measure candidate flow generated by the generation unit 15D. Subsequently, the setting unit 15E executes a smoothing and labeling process on the two-dimensional histogram H1. Accordingly, initial seven clusters of IDs "1" to "7" are obtained. Then, the setting unit 15E calculates an initial value of the number of clusters obtained from the two-dimensional histogram H1 and a representative value of each cluster, for example, an average value. Thereafter, the setting unit 15E executes clustering by fitting a mixed Gaussian distribution and estimating the number of clusters and the distribution of each cluster. Then, the setting unit 15E generates a tree to which the average value (a threshold or a probability) is applied by the number of combinations of the clusters.

By the clustering, the setting unit 15E narrows down the branch threshold and the branch probability, which are candidates to be set in the branch of the measure candidate flow, into clusters.

Further, the branch of the measure candidate flow also includes the condition ID of the OR condition. As described above, in the OR condition branch, the plurality of conditions included in the OR condition are aligned in a specific order. As a mere example, in the condition definition of the condition IDs, a plurality of conditions included in the OR condition can be sorted in ascending order of the parameter type code (see FIG. 8). Further, the condition definition of the condition ID of the branch including the OR condition among the branches of the measure candidate flow can be converted into an expression of an AND condition.

Then, the setting unit 15E calculates a branch parameter that satisfies a target and a constraint condition of the measure, that is, a branch threshold, for the measure candidate flow based on the region data including information such as a resource that can provide a service for each service. A problem of calculating the branch threshold is a problem of dividing a discrete data point sequence into two sets with a certain threshold for each feature, and thus becomes a nonlinear and discontinuous problem. Therefore, by formulating the problem of combination optimization, the branch threshold of each branch of the measure candidate flow can be calculated according to a mathematical optimization algorithm such as a genetic algorithm.

For example, an objective function including a branch threshold in the branch of the measure candidate flow as a variable, for example, an evaluation function, is set. Further, examples of the constraint condition for constraining a range in which a variable can be operated include an "effect target" obtained by multiplication of the number of people allocated to a service and a coefficient of an effect, or the like, a "cost constraint" obtained by multiplication of the number of people allocated to the service and the cost coefficient, or the like, and a "resource constraint" in which a resource that can be provided for each service is set. Under the formulation of the objective function and the constraint condition, a combination of variables that satisfy the constraint condition and optimize the objective function is calculated according to the GA or the like. The branch threshold of each branch of the measure candidate flow is calculated by the combination of the calculated variables.

When the branch threshold of each branch of the measure candidate flow is calculated by such mathematical optimization, the calculation cost is high, so that the branch threshold can be efficiently calculated as follows. That is, it is also possible to calculate a target value $P_d$ of an allocation ratio of an object to the provision-scheduled service for each provision-scheduled service by linear programming or the like based on the above-described effect target and the above-described constraint conditions, and to calculate a branch threshold of each branch of the measure candidate flow based on such a target value of the allocation ratio. For example, the setting unit 15E can optimize the branch threshold of each branch of the measure candidate flow by training a gradient method and a regression model.

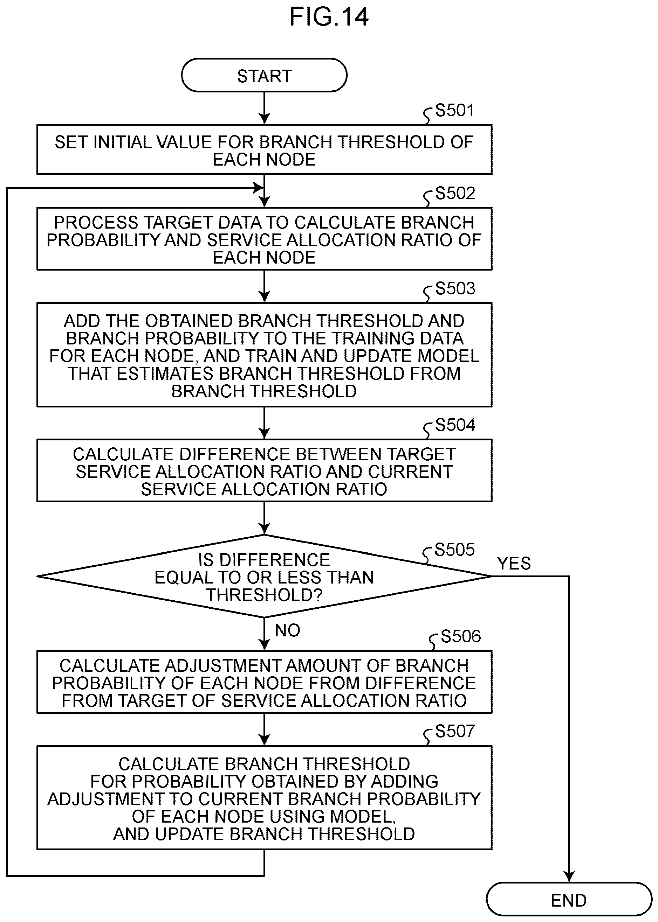

FIG. 14 is a flowchart illustrating a procedure of branch threshold setting process. As illustrated in FIG. 14, the setting unit 15E sets an initial value for the branch threshold of each node of the measure candidate flow (step S501).

Subsequently, the setting unit 15E calculates a branch probability p of each node of the measure candidate flow and an allocation ratio $P_s$ of the provision-scheduled service by applying the target data to the measure candidate flow in which the branch threshold obtained in step S501 or S507 is set (step S502). The "target data" mentioned here is data of a feature amount regarding an object, for example, a resident, belonging to a local government for which a user who makes the above-described measure generation request, for example, a measure planner, is a measure planning target. For example, the target data may be data in which a feature amount for each parameter type used for condition determination in the branch, that is, for each type of feature amount, is associated. By applying the target data to the measure candidate data, the object is finally allocated to the provision-scheduled service at the terminal after the object is allocated to a branch destination corresponding to the feature amount of the object by the branch threshold of the branch node for each branch node. Accordingly, the branch probability p of the branch node is calculated for each branch node, and the allocation ratio $P_s$ to the provision-scheduled service is calculated for each provision-scheduled service.

Here, the allocation ratio and the branch probability of the provision-scheduled service will be described. Focusing on the branch probability of one branch node in the measure candidate flow, the allocation ratio to the provision-scheduled service can be expressed by a linear expression of the following Formula (1). In the following Formula (1), "$u_{j,i}$" and "$v_{j,i}$" are constants. Here, "i" in the following Formula (1) is an index for identifying a branch node included in the measure candidate flow, and "j" in the following Formula (1) is an index for identifying a node of a provision-scheduled service included in the measure candidate flow.

$$P_{sj} = u_{j,i} * p_i + v_{j,i} \qquad \text{Formula (1)}$$

A change in the allocation ratio with respect to a change in the branch probability is obtained by the following Formula (2). "N" in the following Formula (2) indicates the number of branch nodes included in the measure candidate flow and "M" indicates the number of provision-scheduled services included in the measure candidate flow.

$$\frac{\partial \boldsymbol{p}_s}{\partial \boldsymbol{p}} = \begin{bmatrix} \frac{\partial p_{s_1}}{\partial p_1} & \cdots & \frac{\partial p_{s_1}}{\partial p_N} \\ \vdots & \ddots & \vdots \\ \frac{\partial p_{s_M}}{\partial p_1} & \cdots & \frac{\partial p_{s_M}}{\partial p_N} \end{bmatrix} = \begin{bmatrix} u_{1,1} & \cdots & u_{1,N} \\ \vdots & \ddots & \vdots \\ u_{M,1} & \cdots & u_{M,N} \end{bmatrix} \qquad \cdots (2)$$

The branch probability p of each node can be obtained by a gradient descent method with respect to the target value $P_d$ of the allocation ratio $P_s$ of the provision-scheduled service. At this time, differentiation of the loss function L defined by the following Formula (3) can be expressed in the following Formula (4). A differential relationship between a node branch probability and an allocation ratio of a provided service can be obtained by the above Formula (2).

$$L = \frac{1}{2}(\boldsymbol{p}_s - \boldsymbol{p}_d)(\boldsymbol{p}_s - \boldsymbol{p}_d)^T = \frac{1}{2}\sum_{i=1}^{M}(p_s - p_{d_i})^2 \qquad \cdots (3)$$

$$\frac{\partial L}{\partial \boldsymbol{p}_s} = \left[\frac{\partial L}{\partial p_{s_1}}, \cdots, \frac{\partial L}{\partial p_{s_M}}\right] = \left[(p_{s_1} - p_{d_1}), \cdots, (p_{s_M} - p_{d_M})\right] \qquad \cdots (4)$$

From the above Formula (4) and the above Formula (2), the following Formula (5) can be obtained as an update formula of the branch probability of the node. The first term in the following Formula (5) is expressed in the following Formula (6). Further, the second term in the following formula (5) is expressed in the following Formula (7). Here, "α" in the following Formula (5) indicates a training rate.

$$p(t+1) = p(t) - \alpha \cdot \frac{\partial L}{\partial p(t)} \qquad \cdots (5)$$

$$p(t) = [p_1(t), \cdots, p_N(t)], \qquad \cdots (6)$$

$$\frac{\partial L}{\partial p} = \frac{\partial L}{\partial p_s}\frac{\partial p_s}{\partial p} = \left[ \sum_{i=1}^{M}(p_{s_i} - p_{d_i})\frac{\partial p_{s_i}}{\partial p_1}, \cdots, \sum_{i=1}^{M}(p_{s_i} - p_{d_i})\frac{\partial p_{s_i}}{\partial p_N} \right] \qquad \cdots (7)$$

Subsequently, for each node of the measure candidate flow, the setting unit 15E adds the branch threshold and the branch probability obtained in step S502 to the training data, and trains parameters of a regression model that outputs an estimated value of the branch threshold from the branch threshold (step S503). As the regression model, a Gaussian process regression (GPR) model can be used as a mere example.

$$\{\alpha_{i,1} \ldots \alpha_{i,N}\} = \overline{Gpr_i}\left(\{p_{i,1} \ldots p_{i,N}\}\right) \qquad \cdots (8)$$

Then, the setting unit 15E calculates a difference between the target value $P_d$ of the allocation ratio of the provision-scheduled service and the allocation ratio $P_s$ of a current provision-scheduled service calculated in step S502 (step S504).

At this time, when the difference calculated in step S504 is not equal to or less than the threshold (NO in step S505), the setting unit 15E executes the following process. That is, the setting unit 15E calculates an adjustment amount of the branch probability of each node from the difference of the allocation ratio of the provision-scheduled service calculated in step S504 with respect to the target value $P_d$ by the above Formula (7) (step S506).

Then, the setting unit 15E calculates a branch threshold for the probability obtained by adding the adjustment amount to the current branch probability of each node using the regression model, and updates the branch threshold (step S507).

For example, in the GPR model in the above Formula (8), by inputting the branch threshold obtained by adding the adjustment amount calculated in step S506 by the above Formula (5) to the branch threshold calculated in step S502, the input branch threshold is updated by the above Formula (9), and the updated branch threshold is output.

$$\alpha_i \rightarrow \overline{Gpr_i}(p_i^*) \qquad \cdots (9)$$

After the process of step S507 is executed, the process proceeds to step S502. Thereafter, the processes from step S502 to step S507 are repeated until the difference calculated in step S504 becomes equal to or less than the threshold (NO in step S505). When the difference calculated in step S504 is equal to or less than the threshold (YES in step S505), the process ends.

In this way, the setting unit 15E calculates the allocation probability of each node included in the generated measure candidate flow using a machine learning model that outputs an allocation probability of each node included in the measure flow input in response to an input of the measure flow. Further, when the allocation probability of each of the plurality of designated nodes is designated, the setting unit 15E determines a condition of the conditional branch included in the generated measure candidate flow so that an error between the calculated allocation probability and the designated allocation probability becomes small.

The output unit 15F is a processing unit that outputs various types of information to the client terminal 30. As one aspect, the output unit 15F can display the measure candidate flow generated by the generation unit 15D, that is, the instance of the graph structure on the client terminal 30. In addition, the output unit 15F can also cause the client terminal 30 to display the measure candidate flow in which the branch threshold is set by the setting unit 15E at each branch node of the measure candidate flow generated by the generation unit 15D.

<One Aspect of Effect>

As described above, the server apparatus 10 according to the present embodiment extracts, for each service included in the flow graph of the known measures collected in the data infrastructure, an array of conditions corresponding to a path formed by connecting branch nodes up to the terminal node corresponding to the service as a path structure.

Accordingly, the server apparatus 10 according to the present embodiment can implement extraction of a path structure as an example of a factor structure of measures. Further, the measure candidate flow can be generated by retrieving a set of paths corresponding to services scheduled to be provided at the time of implementing the measure in the path structure DB 13B that stores the set of path structures extracted for each service.

Example 2

Although the example related to the disclosed apparatus has been described above, the present invention may be implemented in various different forms other than the above-described embodiments. Accordingly, other examples included in the present invention will be described below.

<Distribution and Integration>

Each constituent of each apparatus illustrated in the drawings is not necessarily physically configured as illustrated in the drawings. That is, a specific form of distribution and integration of each apparatus is not limited to the illustrated form, and all of the constituents can be functionally or physically distributed and integrated in any unit according to various loads, usage conditions, and the like. For example, the acquisition unit 15A, the extraction unit 15B, the reception unit 15C, the generation unit 15D, the setting unit 15E, or the output unit 15F may be connected via a network as an external apparatus of the server apparatus 10. As a mere example, the acquisition unit 15A and the extraction unit 15B, and the reception unit 15C, the generation unit 15D, the setting unit 15E, and the output unit 15F may be implemented by different server apparatuses. The acquisition unit 15A, the extraction unit 15B, the reception unit 15C, the generation unit 15D, the setting unit 15E, or the output unit 15F may be included in different apparatuses which cooperate over a network to implement the functions of the server apparatus 10.

<Hardware Configuration>

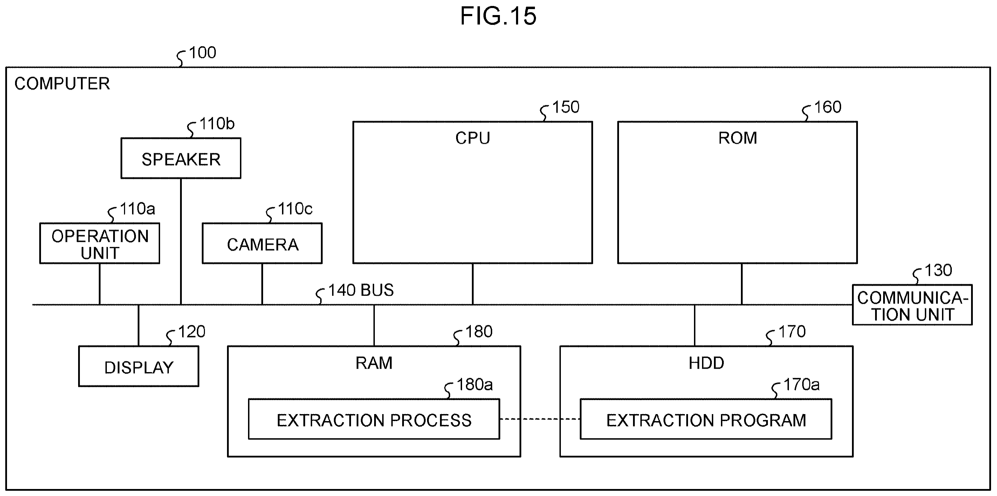

Various processes described in the above examples can be implemented by executing a program prepared in advance on a computer such as a personal computer or a workstation. Accordingly, an example of a computer that executes an extraction program that has the same functions as those of Examples 1 and 2 will be described below with reference to FIG. 15.

FIG. 15 is a diagram illustrating a hardware configuration example. As illustrated in FIG. 15, a computer 100 includes an operation unit 110a, a speaker 110b, a camera 110c, a display 120, and a communication unit 130. The computer 100 further includes a CPU 150, a ROM 160, an HDD 170, and a RAM 180. These units 110 to 180 are connected via a bus 140.

As illustrated in FIG. 15, the HDD 170 stores an extraction program 170a that exhibits functions similar to those of the acquisition unit 15A and the extraction unit 15B described in Example 1. The extraction program 170a may be integrated or separated similarly to the components of the acquisition unit 15A and the extraction unit 15B illustrated in FIG. 1. That is, the HDD 170 does not necessarily store all the data described in the above Example 1, and data used for a process may be stored in the HDD 170.

Under such an environment, the CPU 150 reads the extraction program 170a from the HDD 170 and then loads the extraction program in the RAM 180. As a result, the extraction program 170a functions as an extraction process 180a as illustrated in FIG. 15. In the extraction process 180a, various types of data read from the HDD 170 are loaded in an area allocated to the extraction process 180a in a storage area included in the RAM 180, and various processes are executed using the loaded various types of data. For example, the process illustrated in FIG. 5 is included as an example of the process executed by the extraction process 180a. In the CPU 150, not all the processing units described in the above Example 1 need to operate, and it is sufficient that the processing unit corresponding to a process to be executed is virtually implemented.

The above extraction program 170a does not necessarily have to be stored in the HDD 170 or the ROM 160 from the beginning. For example, each program is stored in a "portable physical medium" such as a flexible disk, a so-called FD, a CD-ROM, a DVD disk, a magneto-optical disc, or an IC card inserted into the computer 100. Then, the computer 100 may acquire and execute each program from the portable physical media. Each program may be stored in another computer, a server apparatus, or the like connected to the computer 100 via a public line, the Internet, a LAN, a WAN, or the like, and the computer 100 may acquire and execute each program from another computer or the server apparatus.

Reference Signs List

| 10 | SERVER APPARATUS |
|---|---|
| 11 | COMMUNICATION CONTROL UNIT |
| 13 | STORAGE UNIT |
| 13A | MEASURE DB |
| 13B | PATH STRUCTURE DB |
| 15 | CONTROL UNIT |
| 15A | ACQUISITION UNIT |
| 15B | EXTRACTION UNIT |
| 15C | RECEPTION UNIT |
| 15D | GENERATION UNIT |
| 15E | SETTING UNIT |
| 15F | OUTPUT UNIT |
| 30 | CLIENT TERMINAL |

**Claims**

1. An extraction method causing a computer to execute processes of:

    acquiring a measure including a plurality of conditional branches coupled by a directed edge and each node coupled to a branch destination of each of the plurality of conditional branches; and
    extracting a conditional branch that is a branch source of each of the nodes from the plurality of conditional branches included in the measure.

2. The extraction method according to claim 1, wherein the extraction process includes a process of extracting an array of conditions corresponding to a path formed by a plurality of conditional branches connected up to a terminal node for each terminal node included in the measure.

3. The extraction method according to claim 2, wherein the extraction process includes a process of extracting a logical value, an operator, and a parameter type defined in the condition from each of the plurality of conditional branches.

4. The extraction method according to claim 2, wherein the computer further executes a process of displaying the array of the conditions in a hierarchical order of a tree structure.

5. The extraction method according to claim 1, wherein, when a plurality of nodes are designated, the computer further executes a process of generating a measure including the plurality of designated nodes and conditional branches corresponding to the plurality of designated nodes with reference to a storage unit that stores each node and a condition of an extracted branch source in association.

6. The extraction method according to claim 5, wherein the computer further executes processes of:

    calculating an allocation probability of each node included in the measure generated in the generation process using a machine learning model that outputs an allocation probability of each node included in the measure input in response to an input of the measure; and

determining a condition of a conditional branch included in the measure generated in the generation process so that an error between the calculated allocation probability and the designated allocation probability becomes small when an allocation probability of each of the plurality of designated nodes is designated.

7. An extraction program causing a computer to execute processes of:

acquiring a measure including a plurality of conditional branches coupled by a directed edge and each node coupled to a branch destination of each of the plurality of conditional branches; and

extracting a conditional branch that is a branch source of each of the nodes from the plurality of conditional branches included in the measure.

8. The extraction program according to claim 7, wherein the extraction process includes a process of extracting an array of conditions corresponding to a path formed by a plurality of conditional branches connected up to a terminal node for each terminal node included in the measure.

9. The extraction program according to claim 8, wherein the extraction process includes a process of extracting a logical value, an operator, and a parameter type defined in the condition from each of the plurality of conditional branches.

10. The extraction program according to claim 8, wherein the computer further executes a process of displaying the array of the conditions in a hierarchical order of a tree structure.

11. The extraction program according to claim 7, wherein, when a plurality of nodes are designated, the computer further executes a process of generating a measure including the plurality of designated nodes and conditional branches corresponding to the plurality of designated nodes with reference to a storage unit that stores each node and a condition of an extracted branch source in association.

12. The extraction program according to claim 11, wherein the computer further executes processes of:

calculating an allocation probability of each node included in the measure generated in the generation process using a machine learning model that outputs an allocation probability of each node included in the measure input in response to an input of the measure; and

determining a condition of a conditional branch included in the measure generated in the generation process so that an error between the calculated allocation probability and the designated allocation probability becomes small when an allocation probability of each of the plurality of designated nodes is designated.

13. An information processing apparatus comprising a control unit configured to execute processes of:

acquiring a measure including a plurality of conditional branches coupled by a directed edge and each node coupled to a branch destination of each of the plurality of conditional branches; and

extracting a conditional branch that is a branch source of each of the nodes from the plurality of conditional branches included in the measure.

14. The information processing apparatus according to claim 13, wherein the extraction process includes a process of extracting an array of conditions corresponding to a path formed by a plurality of conditional branches connected up to a terminal node for each terminal node included in the measure.

15. The information processing apparatus according to claim 14, wherein the extraction process includes a process of extracting a logical value, an operator, and a parameter type defined in the condition from each of the plurality of conditional branches.

16. The information processing apparatus according to claim 14, wherein the control unit further executes a process of displaying the array of the conditions in a hierarchical order of a tree structure.

17. The information processing apparatus according to claim 13, wherein, when a plurality of nodes are designated, the control unit further executes a process of generating a measure including the plurality of designated nodes and conditional branches corresponding to the plurality of designated nodes with reference to a storage unit that stores each node and a condition of an extracted branch source in association.

**18.** The information processing apparatus according to claim 17, wherein the control unit further executes processes of:

calculating an allocation probability of each node included in the measure generated in the generation process using a machine learning model that outputs an allocation probability of each node included in the measure input in response to an input of the measure; and

determining a condition of a conditional branch included in the measure generated in the generation process so that an error between the calculated allocation probability and the designated allocation probability becomes small when an allocation probability of each of the plurality of designated nodes is designated.

FIG.1

# FIG.2

FIG.3

FIG.4

EP 4 734 034 A1

# FIG.5

```
                    ( START )
                        │
                        ▼
        ┌───────────────────────────────────┐
        │       LOOP PROCESS 1              │
        │     NUMBER OF MEASURES K          │
        └───────────────────────────────────┘
                        │
                        ▼
        ┌───────────────────────────────────┐
        │       LOOP PROCESS 2              │
        │  NUMBER OF PATHS L OF k-TH MEASURE │
        └───────────────────────────────────┘
                        │
                        ▼
        ┌───────────────────────────────────┐
        │       LOOP PROCESS 3              │
        │ NUMBER OF BRANCHES M INCLUDED IN PATH l │
        └───────────────────────────────────┘
                        │                    ⌐S101
                        ▼
        ┌───────────────────────────────────┐
        │   EXTRACT CONDITION DEFINITION OF  │
        │      LOGICAL VALUE, OPERATOR,      │
        │ AND PARAMETER TYPE IN m-TH BRANCH  │
        └───────────────────────────────────┘
                        │
                        ▼                    ⌐S102
  NO        ◇ IS CONDITION DEFINITION ◇
  ◄─────────◇     UNNUMBERED?         ◇
  │                    │ YES
  │                    ▼                    ⌐S103
  │         ┌───────────────────────────┐
  │         │   NUMBER NEW CONDITION ID │
  │         └───────────────────────────┘
  │                    │
  └───────────────────►▼                    ⌐S104
        ┌───────────────────────────────────┐
        │ ASSOCIATE BRANCH THRESHOLD AND POSITIVE │
        │ BRANCH PROBABILITY WITH CONDITION ID │
        └───────────────────────────────────┘
                        │
                        ▼
        ┌───────────────────────────────────┐
        │       END LOOP PROCESS 3          │
        └───────────────────────────────────┘
                        │                    ⌐S105
                        ▼
        ┌───────────────────────────────────┐
        │   REGISTER BRANCH SEQUENCE OF PATH l │
        └───────────────────────────────────┘
                        │
                        ▼
        ┌───────────────────────────────────┐
        │       END LOOP PROCESS 2          │
        └───────────────────────────────────┘
                        │
                        ▼
        ┌───────────────────────────────────┐
        │       END LOOP PROCESS 1          │
        └───────────────────────────────────┘
                        │
                        ▼
                    ( END )
```

# FIG.6

f11

n0
eGFR ≤ 30.00
[0.00554]
or
URINARY PROTEIN
≥ 2.00
[0.15312]
p=0.15781

True

False

n2
HbA1c ≥ 6.50
[0.15878]
or
FASTING BLOOD
GLUCOSE ≥ 126.00
[0.04543]
or
DIABETES
TREATMENT = 1.00
[0.29108]
p=0.43073

n1
eGFR ≤ 60.00
[0.09925]
or
URINARY PROTEIN
≥ 2.00
[0.06565]
p=0.15838

True

False

True

False

n6
DIABETIC
NEPHROPATHY
= 1.00
[0.10926]
or
HEALTH GUIDANCE
CATEGORY = 2.00
[0.04138]
p=0.14612

n5
T1
p=0.08983

n4
REMOTE
GUIDANCE
AVAILABLE = 1.00
[0.00000]
p=0.00000

n3
T0
p=0.70881

True

False

True

False

n8
HEALTH GUIDANCE
REQUEST ≠ 0.00
[0.88144]
p=0.88144

n7
T2
p=0.05804

n12
T4
p=0.00000

n11
T3
p=0.13339

True

False

n10
T3
p=0.00875

n9
T0
p=0.00118

# FIG.7

path#1 = [        OR CONDITION

AND CONDITION

[1, ['LE', 0], ['GE',1]],        C11

[1, ['GE', 2], ['GE',3], ['EQ', 4]],        C12

[1, ['EQ', 5], ['EQ', 6]],        C13

[1, ['NE', 7]],        C14

]

path#2 = [

[-1, ['LE', 0], ['GE', 1]],        C21

[1, ['LE', 0], ['GE', 1]],        C22

[-1, ['EQ', 14]],        C23

]

## FIG.8

| eGFR | URINARY PROTEIN | HbA1c | FASTING BLOOD GLUCOSE | DIABETES TREAT- MENT | DIABETIC NEPHRO- PATHY | HEALTH GUIDANCE CATEGORY | DISTRICT CODE | SKIPPING BREAK- FAST | DRINKING FRE- QUENCY |
|---|---|---|---|---|---|---|---|---|---|
| 0 | 1 | 2 | 3 | 4 | 5 | 6 | 8 | 12 | 13 |

## FIG.9

```
┌──────────────┐   ┌──────────────┐        ┌──────────────┐
│  SERVICE #1  │   │  SERVICE #2  │  ···   │  SERVICE #M  │
└──────────────┘   └──────────────┘        └──────────────┘

┌──────────────┐   ┌──────────────┐        ┌──────────────┐
│   PATH #1    │   │   PATH #2    │  ···   │   PATH #K    │
└──────────────┘   └──────────────┘        └──────────────┘
```

seq1

| a1 | -a2 | a3 | ··· | aN |

MEASURE i

| BRANCH THRESHOLD | BRANCH THRESHOLD | BRANCH THRESHOLD |
| POSITIVE BRANCH PROBABILITY | POSITIVE BRANCH PROBABILITY | POSITIVE BRANCH PROBABILITY |

MEASURE k

| BRANCH THRESHOLD | BRANCH THRESHOLD | BRANCH THRESHOLD |
| POSITIVE BRANCH PROBABILITY | POSITIVE BRANCH PROBABILITY | POSITIVE BRANCH PROBABILITY |

# FIG.10

```
        ┌─────────────┐
        │    START    │
        └──────┬──────┘
               │
               ▼                                    ⌐S301
┌──────────────────────────────────────────────────┐
│ RETRIEVE AND REGISTER COMBINATION OF PATHS         │
│ HAVING SAME CONDITION AND DIFFERENT CODES          │
│ IN LIST FOR HEAD ELEMENT OF PATH AND SET i = 1     │
└──────────────────────┬─────────────────────────────┘
                       │
                       ▼                      ⌐S302
              ◇─────────────────────◇   NO
              │  NOT REGISTERED?    │──────────┐
              ◇─────────────────────◇          │
                       │ YES                    │
                       ▼            ⌐S303        │
┌──────────────────────────────────────────────┐ │
│ RETRIEVE COMBINATION OF PATHS HAVING SAME     │ │
│ SEQUENCE OF ELEMENTS UP TO (i)-TH ELEMENT     │ │
│ AND HAVING THE SAME CONDITION AND             │ │
│ DIFFERENT CODES FOR THE (i+1)-TH ELEMENT      │ │
│ WITH RESPECT TO + SIDE PATH OF EACH           │ │
│ COMBINATION REGISTERED IN THE (i)-TH LIST,    │ │
│ AND REGISTER COMBINATION IN LIST              │ │
└──────────────────────┬─────────────────────────┘ │
                       │                             │
                       ▼              ⌐S304           │
              ◇─────────────────────◇   NO           │
              │  NOT REGISTERED?    │─────────────────┤
              ◇─────────────────────◇                 │
                       │ YES                          │
                       ▼            ⌐S305              │
┌──────────────────────────────────────────────┐ │
│ RETRIEVE COMBINATION OF PATHS HAVING SAME     │ │
│ SEQUENCE OF ELEMENTS UP TO (i)-TH ELEMENT     │ │
│ AND HAVING THE SAME CONDITION AND             │ │
│ DIFFERENT CODES FOR (i+1)-TH ELEMENT WITH     │ │
│ RESPECT TO PATH ON ONE SIDE OF EACH           │ │
│ COMBINATION REGISTERED IN THE (i)-TH (i)-TH LIST,│ │
│ AND REGISTER COMBINATION IN LIST              │ │
└──────────────────────┬─────────────────────────┘ │
                       │                             │
                       ▼              ⌐S306           │
              ◇─────────────────────◇   NO           │
              │  NOT REGISTERED?    │─────────────────┤
              ◇─────────────────────◇                 │
                       │ YES                          │
                       ▼            ⌐S307              │
┌──────────────────────────────────────────────┐ │
│                i = i + 1                       │ │
└──────────────────────┬─────────────────────────┘ │
                       │                             ▼
                                              ┌──────────┐
                                              │   END    │
                                              └──────────┘
```

# FIG.11

# FIG.12

# FIG.13

BRANCH
THRESHOLD

BRANCHING
PROBABILITY

| 1 | 5 | 0 | 0 | 8 | 1 | 2 | 0 | 0 | 0 |
|---|---|---|---|---|---|---|---|---|---|
| 0 | 3 | 2 | 0 | 0 | 1 | 0 | 0 | 1 | 0 |
| 0 | 0 | 1 | 1 | 0 | 0 | 0 | 0 | 1 | 0 |
| 0 | 0 | 0 | 0 | 0 | 0 | 2 | 0 | 2 | 0 |
| 5 | 0 | 0 | 0 | 4 | 5 | 0 | 0 | 0 | 0 |
| 0 | 3 | 0 | 0 | 0 | 2 | 0 | 0 | 0 | 0 |
| 0 | 0 | 0 | 0 | 0 | 0 | 0 | 0 | 1 | 2 |
| 0 | 0 | 0 | 3 | 2 | 0 | 0 | 0 | 1 | 5 |

~H1

LABELING

| 1 | 1 |   |   | 2 | 2 | 2 |   |   |   |
|---|---|---|---|---|---|---|---|---|---|
|   | 1 | 1 |   |   | 2 |   |   | 3 |   |
|   |   | 1 | 1 |   |   |   |   | 3 |   |
|   |   |   |   |   |   | 4 |   | 3 |   |
| 5 |   |   |   | 4 | 4 |   |   |   |   |
|   | 5 |   |   |   | 4 |   |   |   |   |
|   |   |   |   |   |   |   |   | 7 | 7 |
|   |   |   | 6 | 6 |   |   |   | 7 | 7 |

CLUSTERING

# FIG.14

START

S501

SET INITIAL VALUE FOR BRANCH THRESHOLD OF EACH NODE

S502

PROCESS TARGET DATA TO CALCULATE BRANCH PROBABILITY AND SERVICE ALLOCATION RATIO OF EACH NODE

S503

ADD THE OBTAINED BRANCH THRESHOLD AND BRANCH PROBABILITY TO THE TRAINING DATA FOR EACH NODE, AND TRAIN AND UPDATE MODEL THAT ESTIMATES BRANCH THRESHOLD FROM BRANCH THRESHOLD

S504

CALCULATE DIFFERENCE BETWEEN TARGET SERVICE ALLOCATION RATIO AND CURRENT SERVICE ALLOCATION RATIO

S505

IS DIFFERENCE EQUAL TO OR LESS THAN THRESHOLD?

YES

NO

S506

CALCULATE ADJUSTMENT AMOUNT OF BRANCH PROBABILITY OF EACH NODE FROM DIFFERENCE FROM TARGET OF SERVICE ALLOCATION RATIO

S507

CALCULATE BRANCH THRESHOLD FOR PROBABILITY OBTAINED BY ADDING ADJUSTMENT TO CURRENT BRANCH PROBABILITY OF EACH NODE USING MODEL, AND UPDATE BRANCH THRESHOLD

END

# FIG.15

COMPUTER 100

SPEAKER 110b

OPERATION UNIT 110a

CAMERA 110c

CPU 150

ROM 160

140 BUS

DISPLAY 120

RAM 180

EXTRACTION PROCESS 180a

HDD 170

EXTRACTION PROGRAM 170a

COMMUNICATION UNIT 130

## INTERNATIONAL SEARCH REPORT

| International application No. |
| --- |
| **PCT/JP2023/023670** |

**A.    CLASSIFICATION OF SUBJECT MATTER**

*G06Q 50/00*(2012.01)i
FI:    G06Q50/00

According to International Patent Classification (IPC) or to both national classification and IPC

**B.    FIELDS SEARCHED**

Minimum documentation searched (classification system followed by classification symbols)

G06Q10/00-99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2020/188731 A1 (NEC CORPORATION) 24 September 2020 (2020-09-24) paragraphs [0020]-[0024] | 1-18 |
| A | JP 2012-248058 A (NIPPON TELEGRAPH AND TELEPHONE CORPORATION) 13 December 2012 (2012-12-13) entire text, all drawings | 1-18 |
| A | JP 2010-176288 A (KABUSHIKI KAISHA TOSHIBA) 12 August 2010 (2010-08-12) entire text, all drawings | 1-18 |

☐ Further documents are listed in the continuation of Box C.    ☑ See patent family annex.

| | |
| --- | --- |
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| **17 August 2023** | **29 August 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/023670**

| Patent document cited in search report | | | Publication date (day/month/year) | Patent family member(s) | Publication date (day/month/year) |
|---|---|---|---|---|---|
| WO | 2020/188731 | A1 | 24 September 2020 | US 2022/0147617 A1 paragraphs [0032]-[0036] | |
| JP | 2012-248058 | A | 13 December 2012 | (Family: none) | |
| JP | 2010-176288 | A | 12 August 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003228647 A